(19) Europäisches Patentamt
European Patent Office
Office européen des brevets

(11) EP 4 235 455 A1

(12) EUROPEAN PATENT APPLICATION

(43) Date of publication:
30.08.2023 Bulletin 2023/35

(21) Application number: 23156045.9

(22) Date of filing: 10.02.2023

(51) International Patent Classification (IPC):
G06F 16/33 (2019.01)

(52) Cooperative Patent Classification (CPC):
G06F 16/3347; G16H 70/00

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 28.02.2022 US 202263314615 P

(71) Applicant: Bayer AG
51373 Leverkusen (DE)

(72) Inventors:
• Santus, Enrico
Hoboken, 07030 (US)

• Bhatti, Aasia
Flanders, 07836 (US)
• Sedghamiz, Hooman
San Diego, 92117 (CA)
• Hogertz, Hendrik
12439 Berlin (DE)
• Hirayama, Juliana Tiemi
05027-000 Sao Paulo (BR)

(74) Representative: BIP Patents
c/o Bayer Intellectual Property GmbH
Alfred-Nobel-Straße 50
40789 Monheim am Rhein (DE)

(54) **SEMANTIC SEARCH TOOL**

(57) The present disclosure relates to the technical area of information retrieval. Aspects of the present disclosure relate to a computer system and a computer-readable medium having instructions for a processor to perform semantic searches, preferably in the field of pharmacovigilance.

EP 4 235 455 A1

## Description

FIELD

The present disclosure relates to the technical area of information retrieval. Aspects of the present disclosure relate to a computer system and a computer-readable medium having instructions for a processor to perform semantic searches, preferably in the field of pharmacovigilance.

BACKGROUND

Pharmacovigilance (PV), also known as Drug Safety Surveillance, is the pharmacologic science relating to the collection, detection, assessment, monitoring, and prevention of adverse effects with pharmaceutical products. This is an important process that allows regulatory authorities to continue to assess benefits and risks throughout the lifecycle of a drug and potentially detect serious adverse events and identify new drug safety signals that were previously undetected. Apart from regulatory requirements, pharmaceutical companies need to engage in pharmacovigilance to serve public health, and to foster a sense of trust with patients who use the drug, and to proactively monitor drug effects.

The process generally involves medical information, which can be received from patients, healthcare providers, medical literature, physicians, pharmaceutical company's sales team, pharmacists, and/or the like. Information collected from different sources needs to be processed in a defined consistent way for electronic submission to the regulatory authorities like FDA (Food and Drug Authority), WHO (World Health Organization), MHRA (Medicines and Health Regulatory Agency), EMA (European Medicines Agency) and other local authorities.

Pharmaceutical companies employ so called safety leaders to process this enormous amount of unstructured data and respond to safety queries related to drug candidates and/or drugs, e.g., from health authorities. Said safety leaders usually use exact search terms and may need to manually search for more specific information about the drug / drug candidate in restricted sources, including adverse event narratives from internal tools (e.g., Oracle's Argus database) and source documents, such as medical reports, e-mails, legal documents, etc. These searches may involve hundreds (or thousands) of documents. Performing these searches manually is not only inefficient, but also inaccurate and prone to error. As a result, important information may not be found.

These and other problems are addressed by the present disclosure.

SUMMARY

The present disclosure provides means for performing semantic searches, in particular in the field of pharmacovigilance.

In a first aspect, the present disclosure provides a computer-implemented method, the method comprising:

- receiving a corpus of text files,

- generating a multitude of semantic vectors from the text files, each of which preferably representing the meaning of a sentence,

- receiving a search query,

- generating a semantic search query vector from the search query,

- identifying text files and/or sections in text files whose semantic vectors have a defined similarity to the search query vector,

- outputting the identified text files and/or sections.

In another aspect, the present disclosure provides a computer system comprising:

a processor; and

a memory storing an application program configured to perform, when executed by the processor, an operation, the operation comprising:

- receiving a corpus of text files,

- generating a multitude of semantic vectors from the text files, each of which preferably representing the meaning of sentence,

- receiving a search query,

- generating a semantic search query vector from the search query,

- identifying text files and/or sections in text files whose semantic vectors have a defined similarity to the search query vector,

- outputting the identified text files and/or sections.

In another aspect, the present disclosure provides a non-transitory computer readable medium having stored thereon software instructions that, when executed by one or more processors of one or more computers, cause the computer(s) to execute the following steps:

- receiving a corpus of text files,

- generating a multitude of semantic vectors from the text files, each of which preferably representing the meaning of sentence,

- receiving a search query,

- generating a semantic search query vector from the search query,

- identifying text files and/or sections in text files whose semantic vectors have a defined similarity to the search query vector,

- outputting the identified text files and/or sections.

Preferred embodiments of the present disclosure can be found in the dependent patent claims, the present description, and in the drawings.

DETAILED DESCRIPTION

The different aspects of the present disclosure will be more particularly elucidated below without distinguishing between the different subject matter of the patent claims (computer-implemented method, computer system, computer-readable storage medium). On the contrary, the following elucidations are intended to apply analogously to all subject matter of the patent claims, irrespective of in which context (method, system, storage medium) they occur.

If steps are stated in an order in the present description or in the claims, this does not necessarily mean that the invention is restricted to the stated order. On the contrary, it is conceivable that the steps can also be executed in a different order or else in parallel to one another, unless one step builds upon another step, this absolutely requiring that the building step be executed subsequently (this being, however, clear in the individual case). The stated orders are thus preferred embodiments of the invention.

As used herein, the articles "a" and "an" are intended to include one or more items and may be used interchangeably with "one or more" and "at least one." As used in the specification and the claims, the singular form of "a", "an", and "the" include plural referents, unless the context clearly dictates otherwise. Where only one item is intended, the term "one" or similar language is used. Also, as used herein, the terms "has", "have", "having", or the like are intended to be open-ended terms. Further, the phrase "based on" is intended to mean "based at least partially on" unless explicitly stated otherwise.

Some implementations will be described more fully hereinafter with reference to the accompanying drawings, in which some, but not all implementations are shown. Indeed, various implementations may be embodied in many different forms and should not be construed as limited to the implementations set forth herein; rather, these example implementations are provided so that this disclosure will be thorough and complete, and will fully convey the scope of the disclosure to those skilled in the art.

The present disclosure provides means for performing semantic searches. In contrast to a classic keyword search, the semantic search not only identifies the text files and/or text sections that contain exact keyword(s), but also those text files and/or text sections that deal with the same subject (in a relation of similarity and/or entailment) as the keyword(s).

The search tool of the present disclosure is usually provided by a computer system. The computer system comprises input means and output means to interact with a user, for example, to receive input and commands from the user and/or to output information to the user.

Preferably, the computer system provides a graphical user interface through which the user can make entries into the computer system and through which information is displayed to the user.

Inputs may be made, for example, using a keyboard, a computer mouse, a stylus, a touch-sensitive display, a microphone, and/or the like. The information may be displayed to the user on a monitor, printed on a printer, output via speakers, and/or the like.

In a first step, the user specifies one or more text files to be searched. The text files are usually stored in one or more databases. A collection of one or more text files is also referred to as a corpus of text files in this disclosure.

The text files can have different formats; they can be, for example, pdf files (pdf: portable document format), word files, excel files, XML files (XML: extensible markup language) and/or other files in which text is stored in a form that allows the recognition of letters and/or words.

Specifying the text files can be done by the user entering names of text files in an input field of the graphical user interface and/or selecting one or more file names and/or file folders from a list of file names and/or file folders.

It is also conceivable that the user drags and drops one or more text files and/or file folders containing one or more text files into an input field of the graphical user interface.

The computer system is configured to receive the user's input regarding the specification of one or more text files and to load the corresponding text file(s) into a working memory of the computer system.

The text(s) in the text file(s) can be preprocessed using common techniques from the field of natural language processing (NLP). Common NLP techniques include normalization and annotation; the former is used to reduce irrelevant surface differences between texts (e.g., capitalization), while the latter enriches the text with relevant information (e.g., adding part-of-speech (pos) tags to words).

Preferably, the normalization process includes acronym resolution, name normalization, date normalization, measurement normalization, and/or capitalization normalization. Preferably, the annotation process includes sentence segmentation, tokenization, pos tagging, and/or dependency parsing.

In a further step, the computer system generates a multitude of semantic vectors from the (preprocessed) text files.

The computer system can be configured to generate one semantic vector for each text file and/or for each page of a multipage text document and/or for each paragraph of a text files and/or for each sentence contained in a text file and/or for each group of a defined number of words contained in a text file and/or for each word contained in a text file.

Preferably, the computer system is configured to generate one semantic vector for each sentence contained in each text file.

For the generation of semantic vectors one or more pre-trained word embedding models can be used. There are word embedding models which were trained on large corpora, which have been shown to learn semantic similarities directly from data, even for specialized biomedical text (see e.g. M. Neumann et al., ScispaCy: Fast and Robust Models for Biomedical Natural Language Processing, arXiv:1902.07669, 2019; I. Beltagy et al.: SciBERT: Pretrained Language Model for Scientific Text, arXiv: 1903.1067; J. Lee et al.: BioBERT: a pretrained biomedical language representation model for biomedical text mining, Bioinformatics 2020, pages 1234-1240).

In a preferred embodiment, feature vectors are generated one at the time by inputting a given sentence into a sentence embedding model such as a Siamese BERT neural network pretrained on the task of Natural Language Inference (specifically, we used the model paraphrase-mpnet-base-v2 from Sentence-BERT library, although other models may be used as well; see, e.g.,: https://www.sbert.net/docs/pretrained_models.html, and N. Reimers, I. Gurevych: Sentence-BERT: Sentence Embeddings using Siamese BERT-Networks, arXiv:1908.10084 [cs.CL]).

Such sentence embedding models are optimized by the training task to generate similar vectors for sentences that hold an entailment relationship (e.g., a premise such as "A man is dreaming on his bad" and a hypothesis such as "The man is sleeping") and different vectors for sentences that do not hold an entailment relation (e.g., "The man is 45 years old"), or that even contradict each other (e.g., "The man is riding a horse").

Using a sentence embedding model to generate semantic vectors has particular advantages. Considering the search query as a premise, we can simply search for those sentences (i.e., hypotheses) that hold an entailment relationship with it by ranking the sentences by their similarity with the premise. Because semantic vectors are nothing more than numerical representations of the corresponding text, in which semantic information is encoded, we can use linear algebraic functions such as dot-product, vector cosine, Euclidean distance and/or other similarity functions to calculate how near (i.e., similar) the vectors are. These metrics return scores that inform on how related the sentences are. For instance, vector cosine returns a score ranging between -1 and +1, where -1 means totally different and +1 totally similar. Most sentences tend to be in between these scores, with the 0 meaning neutral.

Once a text file is processed, its original text, preferably segmented in sentences, can be stored in a relational database together with all the processed results (i.e., normalized and annotated text) and the semantic vectors. The mapping between all the processed pieces, the semantic vectors and the original text can be also stored, to guarantee the translation from one representation to another. All the semantic vectors constitute a matrix against which the similarity of the search vector can be measured.

In a further step, the user inputs and/or selects a search query.

For example, the user can input one or more keywords or a sentence that encodes what the user is looking for (e.g., "patients complained about headaches") in an input field of the graphical user interface.

The user may also select a search query from a list of previously typed search queries.

The computer system is configured to receive the search query from the user, and to generate a semantic vector from the search query.

To better distinguish the semantic vector generated from the search query from semantic vectors generated from the text files, the semantic vector generated from the search query is referred to as (semantic) search query vector in this disclosure.

NLP techniques can be applied to the search query to preprocess the search query, such as normalization and/or annotation (see above).

Preferably, the search query goes through the same preprocessing steps as the text files from the corpus of text files have gone through.

Analogously, the semantic search query vector is preferably generated from the (preprocessed) search query using the same word embedding model(s) as used to generate the semantic vectors from the text files (preferably a sentence embedding model).

The semantic search query vector can be stored in a query table of a database to allow users to repeat the same query multiple times, even on different corpora.

In a further step, text files and/or sections in text files whose vectors have a defined similarity to the search query vector are identified.

For example, the computer system can be configured to compare the search query vector with one or more semantic vectors from the text files.

Preferably, comparing two vectors comprises computing a similarity value for the pair of compared vectors, the similarity value quantifying the degree of similarity between the representations.

The similarity value can, e.g., be a rational number, e.g., from 0 to 1, whereas a similarity value of 0 can mean that there are no similarities between the vectors and a similarity value of 1 can mean that the vectors are identical. It is also possible that the similarity value is a percentage, whereas a percentage of 0 can mean that there are no similarities between the vectors and a percentage of 100% can mean that the vectors are identical. Different quantifications, gradations, and ranges are possible. It is for example possible that the similarity value of two vectors which have no similarities is $-\infty$ whereas the similarity value of two identical vectors is $+\infty$. It is also possible that the similarity value ranges from -1 to 1.

Usually but not necessarily, the greater the match of the vectors and/or the more similar they are, the higher is the similarity value. For the sake of simplicity, the present description assumes that the similarity value is always positive, and the greater the similarity value is, the more similar the two vectors are. However, this is not to be understood as a limitation of the present invention.

For the sake of simplicity, it is assumed for the following explanations that the semantic vectors are $N$-dimensional vectors. For example, the semantic search query vector may be an $N$-dimensional vector $a = [a_1, a_2, \ldots, a_N]$, and a semantic vector of the multitude of semantic vectors from the text files may be an N-dimensional vector $b = [b_1, b_2, \ldots, b_N]$.

The similarity value $s(a, b)$ quantifying the similarity between the two vectors $a$ and $b$ can, e.g., be the Cosine Similarity:

$$s(a,b) = \cos(\theta) = \frac{a \cdot b}{\|a\| \|b\|}$$

in which $\theta$ is the angle between the two vectors $a$ and $b$,

$\|a\|$ is the Euclidean norm of vector $a$ (its length) defined as

$$\|a\| = \sqrt{a_1^2 + a_2^2 + \cdots + a_N^2}$$

and in which $\|b\|$ is the Euclidean norm of vector $b$ (its length) defined as

$$\|b\| = \sqrt{b_1^2 + b_2^2 + \cdots + b_N^2}$$

Mathematically, the Cosine Similarity measures the cosine of the angle $\theta$ between two vectors $a$ and $b$ projected in a multi-dimensional space. The Cosine Similarity captures the orientation (the angle) of each vector and not the length.

In order to include the length of each vector, the Euclidean Distance can be computed:

$$d(a,b) = \sqrt{\sum_{i=1}^{N} (a_i - b_i)^2}$$

Instead of (or in addition to) the Euclidean Distance, other distances can be computed, such as the Manhattan Distance, Chebyshev Distance, Minkowski Distance, Weighted Minkowski Distance, Mahalanobis Distance, Hamming Distance, Canberra Distance, Bray Curtis Distance, or a combination thereof.

The similarity of two (or more) vectors can also be quantified by computing a distance between the two (or more) representations. Usually, the closer the distance, the greater the similarity.

A distance $d(a, b)$ can be converted into a similarity value $s(a, b)$ e.g. by the following equation:

$$s(a,b) = \frac{1}{1 + d(a,b)}$$

In an embodiment, a number $m$ of semantic vectors from the multitude of semantic vectors is identified, each semantic vector of the number $m$ of semantic vectors being more similar to the search query vector than every other semantic vector of the multitude of semantic vectors that does not belong to the number $m$ of semantic vectors. In other words: a number $m$ of semantic vectors that are most similar to the search query vector are identified. The number $m$ is a natural number equal to or greater than 1.

It is also possible to sort the number $m$ of semantic vectors by the magnitude of their similarity value.

In another preferred embodiment, a number $q$ of semantic vectors is identified, each semantic vector of the number $q$ of semantic vectors having a predefined similarity to the search query vector. The number $q$ is a natural number equal to or greater than 0. The identification of semantic vectors having a predefined similarity to the search query vector can, e.g., be done by comparing each similarity value with one or more predefined thresholds.

In case of one threshold, each similarity value can be compared with the predefined threshold, and vectors for which the similarity value is equal to or greater than the predefined threshold can be selected.

In case of two thresholds, e.g., a lower threshold and an upper threshold, each similarity value can be compared with the lower and/or upper predefined threshold, and vectors for which the similarity value is greater than the lower threshold and smaller than the upper threshold can be selected.

Further filter and sorting options are conceivable.

The most similar semantic vectors and/or the semantic vectors with a pre-defined similarity to the search query vector can be stored, e.g., in a data storage. These vectors are referred to as the identified semantic vectors.

In a further step, the text section represented by the identified vectors is determined.

The text section represented by a semantic vector (e.g., a sentence from which the semantic vectors was generated) can be retrieved through the mapping system that was stored in the database and be provided to the user as output.

Preferably, the text whose vector has the greatest similarity to the vector is output first or in first place; the remaining text files and/or text sections follow in the order of decreasing similarity of their semantic vectors with the search query vector.

It is also conceivable that for each text file the number of semantic vectors is determined which have a defined similarity with the search query vector (e.g., a similarity equal to or higher than a minimum similarity). It is conceivable that the text file for which the largest number of semantic vectors with a defined similarity was determined is displayed first or in first place. The remaining text files follow in the order of the number of determined semantic vectors with a defined similarity.

The results of the similarity search can be filtered to exclude any similarity below a predefined threshold. Such a threshold can be determined empirically by an expert to exclude results that did not appear relevant in multiple searches. Preferably, the threshold can be changed by the user if more or less results are desired.

All results can be ranked according to their similarity score, with the results closer to 1 (i.e., the highest similarity) on top.

Additionally or alternatively, other filtering processes can be executed to further increase the precision of the results. Such other filtering processes may include filtering according to other previously used queries, to the presence of some keyword, concepts or values in the text (e.g., measures, drugs, diseases, etc.), etc. Filters include the possibility of filtering according to ontological and taxonomical structures in hyper and hypo classes. For example, headaches can be of multiple types (e.g., migraine, tension-type headache, cluster headache) and users may filter according to only one of these classes.

Text sections whose semantic vector have a defined similarity with the search query vector can be marked (e.g., highlighted in yellow color). In case of color marking, the color value can give an indication of the similarity, e.g., darker yellow for higher similarity, lighter yellow for lower similarity.

Additionally or alternatively, certain aspects of text sections can be marked using different colors (e.g., drug names, diseases, dosages, etc.) so that a user can grasp them more quickly.

In a preferred embodiment, users can manually annotate the results by adding one or more tags at either the sentence or the text level. These tags can be reused across queries, to annotate sentences and documents with respect to some user-relevant topics/goals. For example, a user may find that one result for a query may be relevant to another goal. In this case, the user can simply add a tag referred to the other goal, so that the new result will be available for retrieval when needed.

The search tool is also able to learn from the tagged documents to run implicit queries (i.e., queries that may not be expressed with words). When a user runs multiple queries and tag several results in each of them as relevant to some topics/goals, the search tool can also learn the semantic (i.e., non-textually expressible) commonalities between these results and perform an implicit query to return other results that have some degrees of similarity with the tagged group. The visualization of such results may follow the same format as described above.

The search tool described herein can be used especially in the field of pharmacovigilance.

The operations in accordance with the teachings herein may be performed by at least one computer system specially constructed for the desired purposes or at least one general-purpose computer system specially configured for the desired purpose by at least one computer program stored in a typically non-transitory computer readable storage medium.

The term "non-transitory" is used herein to exclude transitory, propagating signals or waves, but to otherwise include any volatile or non-volatile computer memory technology suitable to the application.

A "computer system" is a system for electronic data processing that processes data by means of programmable calculation rules. Such a system usually comprises one or more "computers", that unit(s) which comprise(s) a processor for carrying out logical operations, and also peripherals.

In computer technology, "peripherals" refer to all devices which are connected to a computer and serve for the control of the computer and/or as input and output devices. Examples thereof are monitor (screen), printer, scanner, mouse, keyboard, drives, camera, microphone, loudspeaker, etc. Internal ports and expansion cards are, too, considered to be peripherals in computer technology.

The term "process" as used above is intended to include any type of computation or manipulation or transformation of data represented as physical, e.g., electronic, phenomena which may occur or reside, e.g., within registers and/or memories of at least one computer or processor. The term processor includes a single processing unit or a plurality of distributed or remote such units.

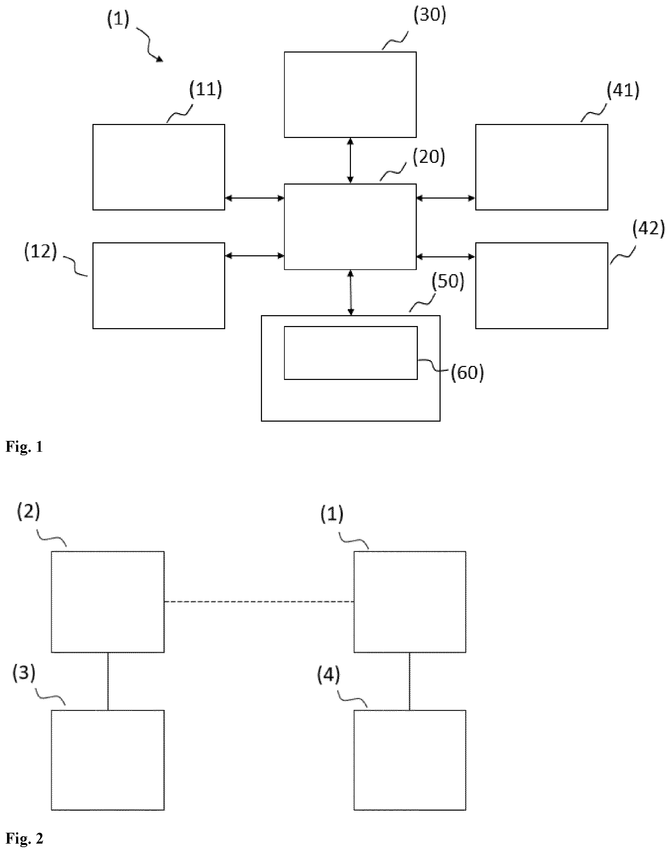

Fig. 1 illustrates a computer system (1) according to some example implementations of the present disclosure in more detail.

Generally, a computer system of exemplary implementations of the present disclosure may be referred to as a computer and may comprise, include, or be embodied in one or more fixed or portable electronic devices. The computer may include one or more of each of a number of components such as, for example, a processing unit (20) connected to a memory (50) (e.g., storage device).

The processing unit (20) may be composed of one or more processors alone or in combination with one or more memories. The processing unit (20) is generally any piece of computer hardware that is capable of processing information such as, for example, data, computer programs and/or other suitable electronic information. The processing unit (20) is composed of a collection of electronic circuits some of which may be packaged as an integrated circuit or multiple interconnected integrated circuits (an integrated circuit at times more commonly referred to as a "chip"). The processing unit (20) may be configured to execute computer programs, which may be stored onboard the processing unit (20) or otherwise stored in the memory (50) of the same or another computer.

The processing unit (20) may be a number of processors, a multi-core processor or some other type of processor, depending on the particular implementation. For example, it may be a central processing unit (CPU), a field programmable gate array (FPGA), a graphics processing unit (GPU) and/or a tensor processing unit (TPU). Further, the processing unit (20) may be implemented using a number of heterogeneous processor systems in which a main processor is present with one or more secondary processors on a single chip. As another illustrative example, the processing unit (20) may be a symmetric multi-processor system containing multiple processors of the same type. In yet another example, the processing unit (20) may be embodied as or otherwise include one or more ASICs, FPGAs or the like. Thus, although the processing unit (20) may be capable of executing a computer program to perform one or more functions, the processing unit (20) of various examples may be capable of performing one or more functions without the aid of a computer program. In either instance, the processing unit (20) may be appropriately programmed to perform functions or operations according to example implementations of the present disclosure.

The memory (50) is generally any piece of computer hardware that is capable of storing information such as, for example, data, computer programs (e.g., computer-readable program code (60)) and/or other suitable information either on a temporary basis and/or a permanent basis. The memory (50) may include volatile and/or non-volatile memory, and may be fixed or removable. Examples of suitable memory include random access memory (RAM), read-only memory (ROM), a hard drive, a flash memory, a thumb drive, a removable computer diskette, an optical disk, a magnetic tape or some combination of the above. Optical disks may include compact disk - read only memory (CD-ROM), compact disk - read/write (CD-R/W), DVD, Blu-ray disk or the like. In various instances, the memory may be referred to as a computer-readable storage medium. The computer-readable storage medium is a non-transitory device capable of storing information, and is distinguishable from computer-readable transmission media such as electronic transitory signals capable of carrying information from one location to another. Computer-readable medium as described herein may generally refer to a computer-readable storage medium or computer-readable transmission medium.

The machine learning model, the trained machine learning model and the segmentation unit may be stored in the memory (50).

In addition to the memory (50), the processing unit (20) may also be connected to one or more interfaces for displaying, transmitting and/or receiving information. The interfaces may include one or more communications interfaces and/or one or more user interfaces. The communications interface(s) may be configured to transmit and/or receive information, such as to and/or from other computer(s), network(s), database(s) or the like. The communications interface may be configured to transmit and/or receive information by physical (wired) and/or wireless communications links. The communications interface(s) may include interface(s) (41) to connect to a network, such as using technologies such as cellular telephone, Wi-Fi, satellite, cable, digital subscriber line (DSL), fiber optics and the like. In some examples, the communications interface(s) may include one or more short-range communications interfaces (42) configured to connect devices using short-range communications technologies such as NFC, RFID, Bluetooth, Bluetooth LE, ZigBee, infrared (e.g., IrDA) or the like.

The user interfaces may include a display (30). The display may be configured to present or otherwise display information to a user, suitable examples of which include a liquid crystal display (LCD), light-emitting diode display (LED), plasma display panel (PDP) or the like. The user input interface(s) (11) may be wired or wireless, and may be configured to receive information from a user into the computer system (1), such as for processing, storage and/or display. Suitable examples of user input interfaces include a microphone, image or video capture device, keyboard or keypad, joystick, touch-sensitive surface (separate from or integrated into a touchscreen) or the like. In some examples, the user interfaces may include automatic identification and data capture (AIDC) technology (12) for machine-readable information. This may include barcode, radio frequency identification (RFID), magnetic stripes, optical character recognition (OCR), integrated circuit card (ICC), and the like. The user interfaces may further include one or more interfaces for communicating with peripherals such as printers and the like.

As indicated above, program code instructions (60) may be stored in memory (50), and executed by processing unit (20) that is thereby programmed, to implement functions of the systems, subsystems, tools and their respective elements described herein. As will be appreciated, any suitable program code instructions (60) may be loaded onto a computer or other programmable apparatus from a computer-readable storage medium to produce a particular machine, such that the particular machine becomes a means for implementing the functions specified herein. These program code instructions (60) may also be stored in a computer-readable storage medium that can direct a computer, processing unit or other programmable apparatus to function in a particular manner to thereby generate a particular machine or particular article of manufacture. The instructions stored in the computer-readable storage medium may produce an article of manufacture, where the article of manufacture becomes a means for implementing functions described herein. The program code instructions (60) may be retrieved from a computer-readable storage medium and loaded into a computer, processing unit or other programmable apparatus to configure the computer, processing unit or other programmable apparatus to execute operations to be performed on or by the computer, processing unit or other programmable apparatus.

Retrieval, loading and execution of the program code instructions (60) may be performed sequentially such that one instruction is retrieved, loaded and executed at a time. In some example implementations, retrieval, loading and/or execution may be performed in parallel such that multiple instructions are retrieved, loaded, and/or executed together. Execution of the program code instructions (60) may produce a computer-implemented process such that the instructions executed by the computer, processing circuitry or other programmable apparatus provide operations for implementing functions described herein.

Execution of instructions by processing unit, or storage of instructions in a computer-readable storage medium, supports combinations of operations for performing the specified functions. In this manner, a computer system (1) may include processing unit (20) and a computer-readable storage medium or memory (50) coupled to the processing circuitry, where the processing circuitry is configured to execute computer-readable program code instructions (60) stored in the memory (50). It will also be understood that one or more functions, and combinations of functions, may be implemented by special purpose hardware-based computer systems and/or processing circuitry which perform the specified functions, or combinations of special purpose hardware and program code instructions.

Fig. 2 shows schematically by way of example a preferred embodiment of the computer system according to the present disclosure.

The computer system comprises a first computer (1) and a second computer (2). The first computer (1) is connected to the second computer system (2) via a network, e.g., the internet.

The first computer system (1) is used by a user. The second computer system (2) provides a graphical user interface to the user via the first computer system (1), for example via a web browser.

Via the first computer system (1), the user specifies one or more text files to be used for a search. The one or more text files can be stored in a data storage (4) connected to the first computer system (1) and/or in a data storage (3) connected to the second computer system (2).

The data storage (4) can be an integral part of the first computer system (1) or it can be separated therefrom and connected to the first computer system (1), e.g., via one or more network connection(s). It is possible that the first computer system (1) has access to more than one data storage.

The data storage (3) can be an integral part of the second computer system (2) or it can be separated therefrom and connected to the second computer system (2), e.g., via one or more network connection(s). It is possible that the second computer system (2) has access to more than one data storage.

The second computer system (2) is configured to receive the one or more text files and to generate a multitude of semantic vectors from the one or more text files.

The word embedding model(s) which is/are used by the second computer system to generate semantic vectors can be stored in the data storage (3).

Via the first computer system (1), the user specifies a search query.

The second computer system (2) is configured to generate a semantic search query vector from the search query.

The second computer system (2) is configured to identify text files and/or sections in text files whose semantic vectors have a defined similarity to the search query vector.

The first computer system (1) is configured to output the identified text files and/or sections to the user.

**Claims**

1. A computer-implemented method, the method comprising:

   - receiving a corpus of text files,
   - generating a multitude of semantic vectors from the text files, each of which preferably representing the meaning of a sentence,
   - receiving a search query,
   - generating a semantic search query vector from the search query,
   - identifying text files and/or sections in text files whose semantic vectors have a defined similarity to the search query vector,
   - outputting the identified text files and/or sections.

2. The method according to claim 1, further comprising:

    - allowing the annotation of the identified text files and/or sections at sentence and/or text level for classifying them according to user-relevant topics and/or goals for future retrieval,
    - learn from the annotation to run implicit queries for identifying semantically related texts to the annotated text files and/or sections.

3. The method according to claim any one of claims 1 or 2, wherein the corpus of text files contains case safety reports related to one or more medicinal products.

4. The method according to any one of claims 1 to 3, wherein the search query contains one or more of the following information: a drug, a drug candidate, an indication, a symptom, an adverse event.

5. The method according to any one of claims 1 to 4, wherein identifying text files and/or sections in text files comprises the steps:

    - computing a similarity value for each semantic vector generated from the text files, the similarity value quantifying the similarity between the respective semantic vector and the semantic search query vector,
    - identifying semantic vectors that have a defined similarity to the search query vector,
    - determining the text files and/or sections from which the identified semantic vectors were generated.

6. The method according to any one of claims 1 to 5, wherein outputting the identified text files and/or sections comprises the steps:

    - ordering the text files and/or sections in the order of decreasing similarity of their semantic vectors to the semantic search query vector,
    - outputting the ordered text files and/or sections, starting with the text file or section with the highest similarity.

7. The method according to any one of claims 1 to 6, wherein outputting the identified text files and/or sections comprises the steps:

    - determining, for each text file, the number of semantic vectors with a defined similarity to the semantic search query vector,
    - ordering the text files according to the number of semantic vectors with the defined similarity to the semantic search query vector,

    - outputting the text files in the order of decreasing number of semantic vectors with the defined similarity to the semantic search query vector, starting with the text file with the highest number.

8. The method according to any one of claims 1 to 7, wherein sections whose semantic vector has a defined similarity to the semantic search query vector are marked and/or highlighted.

9. The method according to any one of claims 1 to 8, wherein the semantic vectors are generated from the text files using a pre-trained word embedding model.

10. The method according to any one of claims 1 to 9, wherein the semantic vectors are generated from the text files using a pre-trained sentence embedding model.

11. The method according to any one of claims 1 to 10, wherein a semantic vector is generated for each sentence of a text file.

12. The method according to any one of claims 1 to 11, wherein the semantic search query vector is generated from the search query by using the same word embedding model used to generate the semantic vectors from the text files.

13. A computer system comprising:

    a processor; and
    a memory storing an application program configured to perform, when executed by the processor, an operation, the operation comprising:

        - receiving a corpus of text files,
        - generating a multitude of semantic vectors from the text files, each of which preferably representing the meaning of a sentence,
        - receiving a search query,
        - generating a semantic search query vector from the search query,
        - identifying text files and/or sections in text files whose semantic vectors have a defined similarity to the search query vector,
        - outputting the identified text files and/or sections.

14. The computer system according to claim 13, wherein the one or more computer(s) are caused to further execute the following steps:

    - allowing the annotation of the identified text files and/or sections at sentence and/or text level for classifying them according to user-relevant topics and/or goals for future retrieval,

- learn from the annotation to run implicit queries for identifying semantically related texts to the annotated text files and/or sections.

15. A non-transitory computer readable medium having stored thereon software instructions that, when executed by one or more processors of one or more computers, cause the computer(s) to execute the following steps:

    - receiving a corpus of text files,
    - generating a multitude of semantic vectors from the text files, each of which preferably representing the meaning of a sentence,
    - receiving a search query,
    - generating a semantic search query vector from the search query,
    - identifying text files and/or sections in text files whose semantic vectors have a defined similarity to the search query vector,
    - outputting the identified text files and/or sections.

16. The non-transitory computer readable medium according to claim 14, wherein the one or more computer(s) are caused to further execute the following steps:

    - allowing the annotation of the identified text files and/or sections at sentence and/or text level for classifying them according to user-relevant topics and/or goals for future retrieval,
    - learn from the annotation to run implicit queries for identifying semantically related texts to the annotated text files and/or sections.

17. Use of the computer system according to any one of claims 13 or 14 and/or the computer readable medium according to any one of claims 15 or 16 for performing searches in the field of pharmacovigilance.

18. Use according to claim 17, wherein the use comprises one or more steps of any one of claims 1 to 12.

(1)

(30)

(11)

(41)

(20)

(12)

(42)

(50)

(60)

**Fig. 1**

(2)

(1)

(3)

(4)

**Fig. 2**

**EUROPEAN SEARCH REPORT**

Europäisches
Patentamt
European
Patent Office
Office européen
des brevets

Application Number

EP 23 15 6045

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/226126 A1 (ZOU JOEL CHI [US] ET AL) 16 July 2020 (2020-07-16) <br> * paragraph [0019] – paragraph [0023]; figure 2 * <br> * paragraph [0024] – paragraph [0026]; figure 3 * <br> * paragraph [0027] – paragraph [0028]; figure 4 * <br> * paragraph [0034]; figure 6 * <br> ----- | 1-18 | INV. <br> G06F16/33 |

TECHNICAL FIELDS
SEARCHED        (IPC)

G06F

The present search report has been drawn up for all claims

| Place of search | Date of completion of the search | Examiner |
|---|---|---|
| Berlin | 31 May 2023 | Polzer, Andreas |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons
........................................................................
& : member of the same patent family, corresponding
  document

EPO FORM 1503 03.82 (P04C01)

## ANNEX TO THE EUROPEAN SEARCH REPORT
## ON EUROPEAN PATENT APPLICATION NO.

EP 23 15 6045

This annex lists the patent family members relating to the patent documents cited in the above-mentioned European search report.
The members are as contained in the European Patent Office EDP file on
The European Patent Office is in no way liable for these particulars which are merely given for the purpose of information.

31-05-2023

| Patent document cited in search report | Publication date | Patent family member(s) | Publication date |
|---|---|---|---|
| US 2020226126 A1 | 16-07-2020 | NONE | |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **M. NEUMANN et al.** ScispaCy: Fast and Robust Models for Biomedical Natural Language Processing. *arXiv:1902.07669,* 2019
- **I. BELTAGY et al.** SciBERT: Pretrained Language Model for Scientific Text. *arXiv: 1903.1067*
- **J. LEE et al.** BioBERT: a pre-trained biomedical language representation model for biomedical text mining. *Bioinformatics,* 2020, 1234-1240
- **N. REIMERS ; I. GUREVYCH.** Sentence-BERT: Sentence Embeddings using Siamese BERT-Networks. *arXiv:1908.10084*